(19) Europäisches Patentamt
European Patent Office
Office européen des brevets

(11) **EP 3 566 642 A1**

(12) **EUROPEAN PATENT APPLICATION**

(43) Date of publication:
**13.11.2019 Bulletin 2019/46**

(51) Int Cl.:
***A61B 5/00*** (2006.01)

(21) Application number: **19158530.6**

(22) Date of filing: **21.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.10.2008 US 19696008 P**
**06.05.2009 US 38773009**

(62) Document number(s) of the earlier application(s) in accordance with Art. 76 EPC:
**09822646.7 / 2 348 965**

(71) Applicant: **ResMed Sensor Technologies Limited Dublin 4 (IE)**

(72) Inventors:
• **Dickinson, David**
**Sudbury, MA 01776 (US)**
• **Donahue, Jason**
**Boston, MA 02118 (US)**
• **Fabregas, Stephen**
**Boston, MA 02118 (US)**
• **Rubin, Benjamin**
**Boston, MA 02118 (US)**
• **Shambroom, John**
**Framingham, MA 01701 (US)**
• **Shashoua, Eric**
**Norwood, MA 02062 (US)**

(74) Representative: **Vossius & Partner**
**Patentanwälte Rechtsanwälte mbB**
**Siebertstrasse 3**
**81675 München (DE)**

Remarks:
•This application was filed on 21.02.2019 as a divisional application to the application mentioned under INID code 62.
•Claims filed after the date of filing of the application (Rule 68(4) EPC).

(54) **DATA-DRIVEN SLEEP COACHING SYSTEM**

(57) The present invention relates to a feedback-based sleep coaching system comprising a sensor configured to measure a physiological signal indicative of an aspect of the user's sleep; at least one local processor located proximately to the sensor and configured to process data received from the sensor, the at least one local processor being configured for communication with a remote processor located remotely from the sensor; and a user interface, wherein the at least one local processor is configured to process the received physiological signal to obtain information of the user's sleep and generate a sleep related advice based on the obtained sleep information, or process the received physiological signal to obtain information of the user's sleep and forward the obtained sleep information to the remote processor for generating a sleep related advice based on the obtained sleep information and receive from the remote processor the sleep related advice, and wherein the user interface is configured to present the generated advice to the user.

FIGURE 2

EP 3 566 642 A1

**Description**

**Cross-Reference to Related Applications**

This application claims the benefit of U.S. Provisional Application No. 61/196,960 filed October 22, 2008 and U.S. Utility Application No. 12/387,730 filed May 6, 2009, which are hereby incorporated by reference herein in their entirety.

**Background**

It is well understood that sleep plays an important role in learning and memory. Despite this, most people tend to not get enough sleep, and when they do sleep, the sleep is often reported to be of poor quality. This lack of high quality sleep may lead to decreased quality of life and decreased performance in critical tasks. Many individuals sleep poorly due to a lack of understanding of the factors that affect their sleep quality, such as sleep hygiene, sleep stages, etc. Current methods and systems to help people get a better night's sleep tend to provide broad recommendations and suggestions that are not personalized for a particular user and therefore not as useful as personalized advice. Even methods that can provide personalized sleep instruction and advice, such as visiting a sleep coach or participating in a sleep study, can be laborious and time-consuming. Thus, there remains a need for systems and methods that improve a person's sleep satisfaction.

**Summary of the Invention**

The systems and methods described herein include more particularly, an easy-to-use, automated sleep coaching system that can provide a personalized sleep coaching plan for a particular user. The systems and methods described herein provide data-driven sleep coaching to a user. In one embodiment, the system comprises a headband-mounted first sensor that senses a first physiological signal associated with a sleeping user, such as an electroencephalogram (EEG). The first sensor may be dry, require no preparation, and be easy to apply with a lightweight headband. The first sensor may transmit the sensed first physiological signal to a first processor such as a base station. The base station may process the received first signal or not, for example by using a Fast Fourier Transform (FFT) to convert the received signal into its constituent frequency bands, but in either case, it transmits the resulting second data set to a second processor such as a host computer. In addition to receiving the second data set from the base station, the host computer may receive one or more indications of user behavior or user characteristics, such as user bedtime, user risetime, or other user sleeping or eating habits. This may be done in the form of a computer-based questionnaire. The host computer may then generate advice for improving user sleep satisfaction such as a sleep coaching plan based at least in part on at least one of the second sleep data set, the one or more indications of user behavior or characteristics, and a database containing sleep-related data and advice. This sleep coaching plan may comprise one or more sleep coaching workshops, which the user may undertake. In one embodiment, the system may also comprise a third processor located remotely from the user, such as a remote server. The third processor mentioned here could be an expert human operator or an automated expert system. The host computer may transmit a third data set based on the second data set to the remote server.

In certain embodiments the second processor may be the remote server. In this case, the remote server may be configured to receive the one or more indications of user behavior or characteristics instead of the host computer, for example through a network or internet interface such as a website. The host computer may act as a way station, forwarding the second data set received from the base station to the remote server through a network or internet interface. The generation of the advice for improving user sleep satisfaction may occur at the remote server instead of at the host computer.

In one embodiment, the first signal, second data set, and third data set may be transmitted via any suitable wireless or wired transmission method, such as radio frequency (RF), infra-red (IR), Bluetooth, WiFi, USB, Ethernet, or other similar interfaces. In one embodiment, the second data set may be transferred via a storage device such as a portable USB flash drive, a Secure Digital (SD) card, or other similar storage devices.

In certain embodiments, the first processor and the second processor may be located in the same housing. For example, a personal computer may act as both the base station, or first processor, and the host computer, or second processor. In another embodiment, the remote server may act as the first and second processor, and be located at a central location geographically remote from the user.

In certain embodiments, the first processor may display the first signal to the user on a display such as a television, computer monitor, or other similar display. The display may be in the same housing as the first processor. The first signal may be displayed in a form such as a hypnogram. In one embodiment, the display may also display data such as the current time. Similarly, the generated advice for improving user sleep satisfaction may be displayed to the user on a display such as a television, computer monitor, or other similar display. In one embodiment, the generated sleep-related recommendation may be displayed to the user on a website accessible on a network, such as a local area network (LAN), wide area network (WAN), or the Internet. In another embodiment, the generated sleep-related recommendation may be displayed to the user by sending an email accessible on a network, such as a local area network (LAN), wide area network (WAN), or the Internet.

The first or second processors may have a user interface. The user interface may be a remote control, a keyboard, a touchscreen, or other similar interface.

The user behavior or characteristics may comprise at least one of age, gender, sleeper type/subtype, sleep hygiene, and sleep diary.

One or more sleep coaching workshops may comprise personalized advice generated based at least on the first set of sleep data, such as a recommended bed time, or a limit on caffeine consumption. In certain embodiments, a sleep coaching workshop may relate to a specific user sleep-related issue identified from gathered user sleep or behavior data. User sleep-related issues may comprise issues such as difficulty falling asleep after consumption of caffeine or difficulty staying asleep after consumption of alcohol. In certain embodiments, a sleep coaching workshop may comprise a user questionnaire related to a specific user sleep-related issue, one or more pieces of sleep-related advice, and a summary of results generated based on user sleep performance during the workshop. Sleep-related advice may comprise advice such as abstaining from caffeine or alcohol after noon, or refraining from exercising several hours before bedtime. The summary of results may comprise sleep parameter changes resulting from adoption of a piece of sleep-related advice, such as improved user sleep satisfaction resulting from abstention from caffeine. Sleep satisfaction could be based on objective changes in sleep data or be based on a user's subjective assessment of their own sleep.

In one aspect, the invention provides a kit for an interactive sleep coaching program. The kit comprises a sleep sensor of the type that measures a physiological signal and generates and displays sleep data that characterizes a user's sleep. The kit further comprises a sleep coaching program for collecting information about the user's sleeping conditions and for selecting as a function of an algorithm that considers the collected information, a targeted set of advice stored within a data base of stored advice, for improving the sleep satisfaction of the user, whereby the user may collect advice from the sleep coaching program and employ the sleep sensor to determine interactively whether the advice and sleep coaching program are improving their sleep satisfaction.

In certain embodiments, the sleep coaching program includes means for collecting user data respective of at least one of demographic data and lifestyle data. Optionally, the sleep coaching program includes means of collecting data representative of the user sleep data. In certain embodiments, the sleep coaching program includes means for collecting data representative of user goals for improving sleep satisfaction and employs these goals when selecting advice.

In certain embodiments, the sleep coaching program collects data from the sensor representative of a baseline measure of user sleep quality. Optionally, the sleep coaching program generates an assessment of changes in sleep quality as a function of a previous measure of sleep data and subsequent measures of user sleep data. In certain embodiments, the sleep coaching program generates periodic assessments as a function of milestones within the sleep coaching program, a measured baseline of user sleep quality, and/or a normalized baseline representative of a normative sleep quality measure of a predetermined population. Optionally, the sleep coaching program allows the user to enter sleep data for providing feedback to the sleep coaching program to select subsequent advice from the data base and/or collects diary data from the user representative of events in the user's life over a selected time period that affect the user's sleeping conditions. In all of the above embodiments, the kit may further include means for communicating with a live sleep coach and exchanging sleep data of the user and receiving expert advice from the live sleep coach.

In another aspect, the invention provides an interactive sleep coaching system. The interactive sleep coaching system comprises a sensor of the type that can be worn by a user to measure a physiological signal to collect user sleep data and a table-top processor unit for communicating with the sensor and recording the sleep data collected by the sensor over a defined period of time. The table-top processor unit includes a baseline processor for generating a baseline representative of sleep quality of the user. The interactive sleep coaching system further comprises a user data input device for collecting diary data indicative of events in the user's life and the timing of those events, a processor for correlating, at least as a function of time, the recorded sleep data with the collected diary data to generate a first set of advice for improving the sleep satisfaction based at least in part on the sleep data associated with the defined period of time, and a progression processor for collecting sleep data over a second later period of time and providing to the user a second set of sleep advice for improving the sleep satisfaction based at least in part on the sleep data associated with the second later period of time and the first set of advice.

In certain embodiments, the progression processor includes means for adjusting the baseline as a function of sleep data collected over the second alter period of time, to revise the baseline to reflect changes in sleep over time.

In yet another aspect, the invention provides a method for providing an interactive sleep coaching program to a user. This method includes receiving sleep data associated with a first day sleep data associated with a second day and being indicative of quality of sleep, wherein the sleep data is determined by sensing and processing a physiological signal of the user while the user is sleeping. This method also includes receiving diary data indicative of user lifestyle events, the diary data including data received from the user describing lifestyle events during the first day and data received from the user describing lifestyle events during the second day. This method further includes mapping the sleep data associated with the first day to the diary data associated with the first day, providing to the user a first set of advice for improving user sleep satisfaction based at least in part on the sleep data associated with the first day, mapping the sleep data associated with the second day to the diary data associated with the second day, and providing to the user a second set of advice for improving user sleep satisfaction based at least in part on the sleep data associated with the second day and the first set of advice.

In all of the above aspects and embodiments, the physiological signal may be an electroencephalogram or electroencephalogram signal. The physiological signal may also be movement, respiration, heart rate, heart rate variability, peripheral arterial tone, galvanic skin response, temperature, etc. In all of the above aspects and embodiments, the first set of advice for improving user sleep satisfaction may include a sleep coaching plan. The sleep coaching plan includes at least one sleep coaching workshop directed to at least one sleep-related issue generated based at least in part on at least one of the first physiological signal and the indication of user behaviors or user characteristics. The at least one sleep coaching workshop includes a questionnaire, at least one piece of advice to improve user sleep quality, and a summary of results based at least in part on the first physiological signal received during the workshop.

The following aspects are preferred embodiments of the invention.

1. A kit for an interactive sleep coaching program, comprising
a sleep sensor of the type that measures a physiological signal and generates and displays sleep data that characterizes a user's sleep; and
a sleep coaching program for collecting information about that respective user's sleeping conditions and for selecting as a function of an algorithm that considers the collected information, a targeted set of advice stored within a database of stored advice, whereby the user may collect advice from the sleep coaching program and employ the sleep sensor to determine interactively whether the advice and sleep coaching program are changing their sleep characteristics.

2. The kit of aspect 1, including a processing unit for executing the sleep coaching program.

3 The kit of aspect 1, wherein the sleep coaching program includes
means of collecting data representative of the user sleep data or means for collecting user data respective of at least one of demographic data and lifestyle data.

4. The kit of aspect 1, wherein the sleep coaching program includes
means for collecting data representative of user goals for improving sleep satisfaction and employs goals when selecting advice.

5. The kit of aspect 1, wherein the sleep coaching program
collects data from sensor representative of a baseline measure of user sleep.

6. The kit of aspect 1, wherein the sleep coaching program
generates an assessment of changes in sleep quality as a function of a previous measure of sleep data and subsequent measures of user sleep data.

7. The kit of aspect 1 wherein the sleep coaching program allows the user to select a program for improving sleep satisfaction and selects the advice as a function of the user selected program.

8. The kit of aspect 1, wherein the sleep coaching program generates assessments as a function of milestones within the sleep coaching program.

9. The kit aspect of 8, wherein the sleep coaching program generates the assessments as a function of a measured baseline of users sleep.

10. The kit aspect of 8, wherein the sleep coaching program generates the assessments as function of a normalized baseline representative of a normative sleep measure of a predetermined population.

11. The kit of aspect 1, wherein the sleep coaching program allows the user to enter sleep data for providing feedback to the sleep coaching program to select subsequent advice from the data base.

12. The kit of aspect 1, wherein the sleep coaching program collects diary data from the user representative of events in the user's life over a selected time period that affect the user's sleeping conditions.

13. The kit of aspect 1, further comprising
means for communicating with a live sleep coach and exchanging sleep data of the user and receiving expert advice from the live sleep coach.

14. An interactive sleep coaching system, comprising:

a sensor of the type that can be worn by a user to measure a physiological signal to collect user sleep data,
a processing unit for communicating with the sensor and recording the sleep data collected by the sensor over a defined period of time, having a baseline processor for generating a baseline representative of sleep quality of the user,
a user data input device for collecting diary data indicative of events in the user's life and the timing of those events,
a processor for correlating, at least as a function of time, the recorded sleep data with the collected diary data to generate a first set of advice for improving the sleep satisfaction based at least in part on the sleep data associated with the defined period of time, and
a progression processor for collecting sleep data over a second later period of time and providing to the user a second set of sleep advice for improving the sleep satisfaction based at least in part on the sleep data associated with the second later period of time and the first set of advice.

15. The system of aspect 14, wherein the progression processor includes
means for adjusting the baseline as a function of sleep data collected over the second later period of time, to revise the baseline to reflect changes in sleep over time.

16. The system of aspect 14, wherein the physiological signal is an electroencephalogram signal.

17. The system of aspect 14, wherein the first set of advice for improving user sleep satisfaction comprises
a sleep coaching plan comprising
at least one sleep coaching workshop directed to at least one sleep-related issue generated based at least in part on at least one of the first physiological signal and the indication of user behaviors or user characteristics, wherein the at least one sleep coaching workshop comprises

a questionnaire;
at least one piece of advice to improve user sleep satisfaction; and
a summary of results based at least in part on the first physiological signal received during the workshop.

18. A method for providing an interactive sleep coaching program to a user, comprising
receiving sleep data associated with a first day and being indicative of quality of sleep, wherein the sleep data is determined by sensing and processing a physiological signal of the user while the user is sleeping,
receiving diary data indicative of user lifestyle events, the diary data including data received from the user describing lifestyle events during the first day,
mapping the sleep data associated with the first day to the diary data associated with the first day,
providing to the user a first set of advice for improving user sleep satisfaction based at least in part on the sleep data associated with the first day,
receiving sleep data associated with a second day and being indicative of quality of sleep, wherein the sleep data is determined by sensing and processing a physiological signal of the user while the user is sleeping,
receiving diary data indicative of user lifestyle events, the diary data including data received from the user describing lifestyle events during the second day,
mapping the sleep data associated with the second day to the diary data associated with the second day, and
providing to the user a second set of advice for improving user sleep satisfaction based at least in part on the sleep data associated with the second day and the first set of advice.

19. The method of aspect 18, wherein the physiological signal is an electroencephalogram signal.

20. The method of aspect 18, wherein the first set of advice for improving user sleep satisfaction comprises
a sleep coaching plan comprising
at least one sleep coaching workshop directed to at least one sleep-related issue generated based at least in part on at least one of the first physiological signal and the indication of user behaviors or user characteristics, wherein the at least one sleep coaching workshop comprises

> a questionnaire;
> at least one piece of advice to improve user sleep satisfaction; and
> a summary of results based at least in part on the first physiological signal received during the workshop.

**Brief Description of the Drawings**

The invention may be better understood from the following illustrative description, taken in conjunction with the accompanying drawings in which:

> **FIG. 1A** shows an exemplary data driven sleep coaching system, according to an illustrative embodiment of the invention;
> **FIG. 1B** shows an alternative data driven sleep coaching system, according to an illustrative embodiment of the invention;
> **FIGS. 2** and **3** are block diagrams of an exemplary data driven sleep coaching system, according to an illustrative embodiment of the invention;
> **FIG. 4** shows an exemplary hypnogram, according to an illustrative embodiment of the invention;
> **FIG. 5** is a flow chart of steps involved in an exemplary sleep coaching program, according to an illustrative embodiment of the invention; and
> **FIG. 6** is a flow chart of steps involved in an exemplary method for generating sleep-related advice to improve user sleep quality, according to an illustrative embodiment of the invention.

**Detailed Description of Illustrative Embodiments**

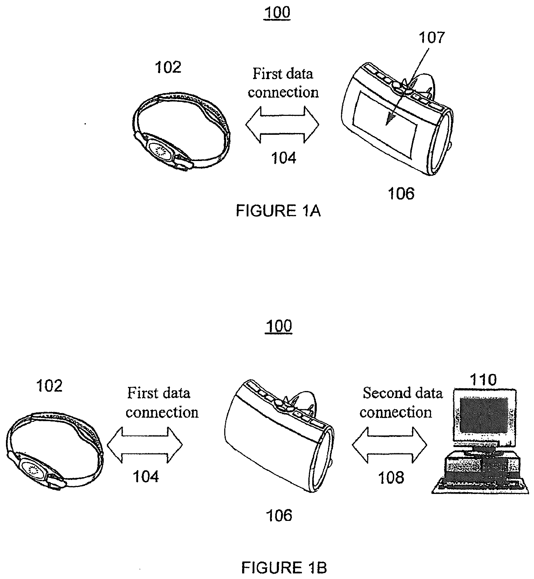

**FIG. 1A** depicts an exemplary data-driven sleep coaching system **100** comprising three modules, according to an illustrative embodiment. A first sensor **102** may be linked to a base station **106** via a first data connection **104**. In an alternative data-driven sleep coaching system shown in **FIG. 1B**, the base station **106** may optionally be linked to a host computer **110** via a second data connection **108**. In one embodiment, the second data connection **108** may involve a portable memory device such as a Secure Digital (SD) media card or a USB flash drive to transfer data from the base station **106** to the host computer **110**.

The sensor **102** may have electrodes or other sensors for sensing one or more user physiological signals. In one embodiment, the sensor **102** has at least one electrode for sensing an electroencephalogram (EEG). In certain embodiments, sensor **102** may have one or more sensors for sensing one or more of electroencephalograms, electrooculograms, electromyograms, pulse rate, respiration rate, body movement, or any other user physiological signal. In certain embodiments, sensor **102** may be in the form of a flexible or rigid band that may be fastened around some portion of the user, such as the wrist, ankle, waist, or head. A sensor **102** that is worn by the user may include soft flexible head bands, such as the depicted sensor **102** of FIGS. 1A and 1B. In one particular embodiment, the sensor **102** includes one or more soft electrically conductive biosensors that may make contact with the patient's skin. The user may tighten the head band so that the soft conductive sensors are put in contact with the user's skin, with the contact being sufficient to all the electrical conductive sensors to record electro-physiological signals, or any suitable signal of the user.

Accordingly, FIGS. 1A and 1B depict an interactive sleep coaching system that has a head band sensor **102** that the user wears to allow the system 100 to measure a physiological signal, such as an EEG signal. The measured physiological signals may be analyzed or otherwise processed to collect user sleep data. For example, in the embodiment having an EEG head band sensor, the sensor **102** measures an EEG signal. Measured EEG signal is passed to the processor **106**, which in this embodiment is a table top bedside unit. As depicted by the data exchange arrow **104**, the sensor **102** and the depicted table top processor **106** exchange data. The data exchange is sufficient to at least transmit data representative of the measured physiological signal from the head band sensor 102 to the depicted table top bedside processor unit **106.** In other embodiments, sensor **102** may include one or more non-contact sensors that may be able to sense user physiological signals. Exemplary sensor modules are further described in U.S. Application Nos. 11/586,196 filed October 24, 2006, 11/499,407 filed August 4, 2006, and 11/069,934 filed February 28, 2005, the entireties of which are hereby incorporated by reference herein.

In certain embodiments, the base station **106** may include a display **107**. Display **107** may be used to display information to the user, or may be used by the user in conjunction with a user interface (not shown) to provide information to the base station **106**. In certain embodiments, display **107** may be a touchscreen display, and the user interface may be integrated into the display **107**.

The base station **106** and the host computer **110** may optionally reside in the same housing (not shown). For example, a personal computer may act as both the base station **106** and the host computer **110**.

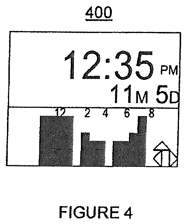

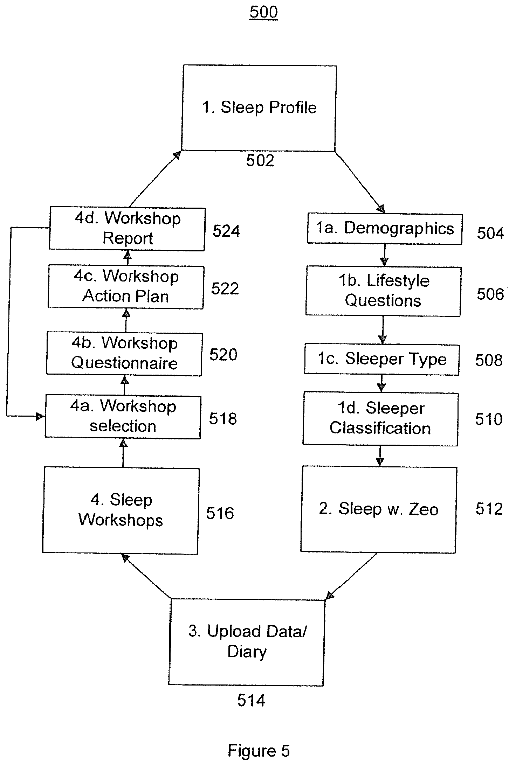

In such an embodiment, the processor unit **106** includes a conventional data memory for storing the recorded physiological signal. The process unit **106** also includes a programmed microprocessor or other data processing device for processing the raw physiological signal to generate sleep data. To this end, the processor unit 106 processes the measured physiological signal to generate a set of metrics that quantitatively measure physical characteristics of the user's sleep event, where the sleep event is a defined sleeping event, such as a night of sleep or an daily nap. For example, the processor unit **106** may process the physiological signal to determine a time at which the user started to sleep and a final time representation of when the user stopped sleeping for a defined sleep event, such as the sleep events that occurred during the night hours or during some other defined periods of time. In one particular embodiment, the process unit 106 includes a baseline processor for generating a baseline measure representative of sleep quality of the user. The baseline measure may be determined as described with reference to FIGS. 4 and 5 and optimally displayed to the user. In this way, the system 100 gives the user feedback representative of the quality of their sleep.

In this optional embodiment where the system 100 is incorporated into a personal computer, the processor unit 106 has a user interface that allows a user to answer survey questions about their current physical conditions, such as their age, gender, and general health. The user can enter additional information, such as information about their stress levels, or the hours that they typically work during the day or week. The user can enter information about their sleep habits, note specific habits, describe their sleeping environment, such as whether they have a sleeping partner, or room darkening shades, or note events surrounding their sleep. Further optionally, they can also keep a sleep diary. The sleep diary would collect information about a users consumption before bed on a particular day, their anxiety level on that same day, and whether they remember being dist///urbed by a bed partner that night. The survey and optional sleep diary information provide information about physical and psychological characteristics of the user.

Optionally, the processor 106 may have a database of stored information, typically advice, for improving the user's sleep satisfaction. The database may be any suitable database and the processor 106 will have a database management system that allows data stored within the database to be selected and presented to the user.

The database can be accessed by a sleep coaching computer program that analyzes the information about that respective user's sleeping conditions and selects from the database advice that is tailored to the user's particular characteristics. To this end, the processor 106 selects the advice by operation of an algorithmic process that considers the survey information about the user. In this way, the user is presented with targeted advice selected by the process to address their situation. As an example, a user who sleeps with a bed partner who disturbs their sleep would be given advice to cope with this difficulty, where a user who sleeps in bed alone would be given different advice. Optionally, as will be discussed in more detail below, the system can select advice based on the sleep data and the user characteristics determined from the user survey.

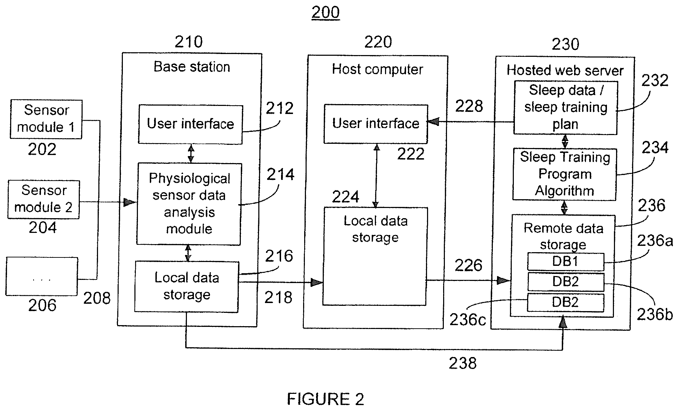

FIG. 2 is a functional block diagram of an exemplary data-driven sleep coaching system with a remote server component. One or more sensor modules **202**, **204**, and/or **206** may be linked to a base station **210** via a first data connection **208**, which may be any type of wired or wireless connection known to those skilled in the art, such as radio frequency (RF), Bluetooth, WiFi, infra-red, wired USB, Ethernet, serial, or other similar interfaces. The sensor modules **202**, **204**, and/or **206** may be configured to sense one or more user physiological signals, which may then be transmitted to base station **210**. The sensor modules **202**, **204**, and/or **206** may be further configured to condition the sensed physiological signals before transmission to base station **210**.

Base station **210** may have a user interface **212**, a sensor data analysis module **214**, and local data storage **216**. User interface **212** may include user input devices such as a keyboard, a touchscreen, an array of buttons, or a radio frequency or infra-red link to a remote control input device. User interface **212** may also include devices for communicating data to the user visually and/or audibly, such as a display screen or a speaker. Sensor data analysis module **214** may be configured to receive data from one or more sensor modules **202**, **204**, and/or **206** from data connection **208** and/or the user interface **212**. Sensor data analysis module **214** may generate a first set of sleep data indicative of quality of sleep from the sensor data received via data connection **208** by converting sensor data into data that may represent metrics of sleep quality and quantity and may be more compact in memory footprint. Sleep data may be collected from monitoring the user. The sleep data typically includes a set of metrics that quantitatively measure physical characteristics of the user's sleep event, where the sleep event is a defined sleeping event, such as a night of sleep or an daily nap. The metrics that can be used by the coaching systems and methods described herein are illustrated and described with, among other places, reference to FIGS. 4 and 5.

In one embodiment, the sensor data may be raw EEGs. The sensor data analysis module may use a digital processing mechanism such as Fast Fourier Transform (FFT) to convert the raw EEG data into its constituent frequency bands. Then a neural net approach may be used to convert the frequency band information into stages of sleep on an epoch by epoch basis, where each epoch may be a slice of time from 30 seconds to 2 minutes long. In certain embodiments, the sensor data analysis module may also generate and store a first set of sleep parameters representing user sleep quality, such as total time spent sleeping, the breakdown of time spent in various stages of sleep, and the computation of a single sleep score to represent the quality of sleep. The sleep stage at each epoch may be stored in the form of a hypnogram. The user interface **212** may be used to present this information to the user for instant feedback.

The received and generated data may be stored in local data storage **216.** Local data storage **216** may be physical memory embedded within base station **210** which may include, but is not limited to, one or more hard drives or random access memory (RAM), or a portable memory device which may include, but is not limited to, SD cards, mini SD cards, micro SD cards, XD cards, CompactFlash memory, Memory Stick, Memory Stick Duo, or any other such types of miniaturized portable memory devices. This stored data may then be transmitted to host computer **220** via second data connection **208**. In one embodiment, the second data connection **208** may involve a wireless interface between the base station and the computer. The wireless interface may involve a standard radio frequency link, where a radio frequency dongle may be plugged into the host computer via a standard input/output port such as a USB port. A proprietary protocol may be used to transmit the sleep data from the base station **210** to the host computer **220**. Other wireless protocols may be used, such as Bluetooth® wireless technology, WiFi, infra-red, or other standard wireless data transport mechanisms.

In certain embodiments, the second data connection **208** may involve a wired interface between the base station **210** and the host computer **220**. The wired interface may utilize a standard port on the computer, such as the USB port, the firewire port, the parallel port, or other types of data ports for data uploading.

In certain embodiments, a portable memory device may be utilized to store the data within the base station **210**. For example, the portable memory device may be plugged into a receptacle in the base station **210** for data capture over several nights. This portable memory device may then be extracted and plugged into a card reader that is connected to the host computer **220** for data uploading. Suitable portable memory devices may include, but are not limited to, SD cards, mini SD cards, micro SD cards, XD cards, CompactFlash memory, Memory Stick, Memory Stick Duo, or any other such types of miniaturized portable memory devices. In yet another embodiment, the portable memory device might involve a standard USB "thumb drive". The thumb drive might be plugged into a receptacle on the base station **210** for several nights to record data. It might then be removed and plugged into a standard USB port on host computer **220** for data uploading.

In any of the above embodiments for the second data connection **208,** the host computer **220** may serve as a way station for the sleep data. It may utilize an internet connection to forward this data to a hosted web server **230,** where the data may be stored in remote data storage **236** and used by a web based application for the generation of personalized sleep coaching tips and tricks for the user.

In another embodiment for the second data connection **208,** the processed sleep data on the base station **210** may bypass the host computer **220** altogether, and may be uploaded directly to the hosted web server **230** via a wired or wireless internet connection **238**. For example, the base station **210** may be plugged physically into a router via an Ethernet cable, or it may communicate wirelessly with a WiFi router. Alternatively, the base station **210** may be equipped with a radio that utilizes a wide area network for data upload via a cellular protocol such as GPS/GPRS, EDGE, UMTS, HSDPA, CDMA, EVDO, WIMAX and the like.

Once the data is uploaded to the hosted web server **230**, the data may be fed into a processor running a Sleep coaching Program (SCP) Algorithm **234**, which may analyze the data and generate a first set of sleep parameter changes for improving user sleep satisfaction. In addition to the processed sleep data, the user may also use a user interface **212** or **222** to answer survey questions about their sleep habits, note specific habits or events surrounding their sleep, and to keep a sleep diary. The survey and optional sleep diary information provide information about physical and psychological characteristics of the user. The SCP algorithm **234** takes all this information into consideration to generate an interactive sleep coaching program or first set of advice for improving user sleep satisfaction in the form of a set of customized, step by step instructions **232**, with the object of coaching the user to improve his or her sleep satisfaction over time. In certain embodiments, the user-provided sleep behavior and characteristics may be stored in a first computer memory database **236a** in remote data storage **236**. In certain embodiments, the first database may be located in local storage **216**, **224**, or at any other location with storage capabilities.

In certain embodiments, a second computer memory database **236b** may store sleep-related data such as information relating sleep parameters or sleep parameter changes to quality of sleep. For example, second database **236b** may contain information about optimal sleep requirements as a function of age, information relating consumed caffeine quantities to sleep parameters, information about the effects of increasing deep sleep time on total sleep quality, and other data relating sleep parameters, sleep parameter changes, or user behavior to sleep quality. In certain embodiments, a third computer memory database **236c** may store sleep-related advice such as advice for improving user sleep satisfaction. For example, third database **236c** may contain information and advice about reducing caffeine or alcohol consumption to improve sleep satisfaction, such as the amount of caffeine or alcohol consumption allowable before adverse effects are seen in sleep parameters or daytime subjective or objective parameters, or how long before bedtime caffeine or alcohol consumption should be stopped for improved sleep satisfaction. User sleep quality may be measured in terms of sleep-related parameters such as the ZQ factor, calculated as shown below. In certain embodiments, second database **236b** and third database **236c** may be located in remote data storage **236**. In other embodiments, second database **236b** and third database **236c** may be located in local storage **216**, **224**, or any other location with storage capabilities. In certain embodiments, second database **236b** and third database **236c** are located in different storage areas.

In certain embodiments, the first, second and third databases may be combined into at least one database. This at least one database may be stored at the base station **210**, the host computer **220**, the web server **230**, or any other location with storage capabilities.

In any of the above embodiments, the sleep coaching program algorithm may use data from the first, second, and third databases to generate the interactive sleep coaching program.

The Graphical User Interface (GUI) for the sleep coaching program algorithm **234** may be displayed via a web browser on user interface **222**, where pertinent sleep data may be presented to the end user utilizing specific user interface constructs that make it easy for end users to understand sleep data.

In an exemplary embodiment, the user uses a secure login mechanism to access his or her personal sleep data hosted on the web server **230**. All the data is centralized on the server **230** and backed up routinely. This implementation may allow the user to access his or her own data, as well as access a variety of community tools, such as a sleep forum, on line chat with a sleep coaching professional, and a variety of other features available over the internet.

In addition to physiological variables and lifestyle factors, environmental cues may also be tracked over the course of time as the environment may have an effect on a person's sleep. These factors may be tracked automatically using sensors (not shown in figure) within the sensor modules **202**, **204**, and/or **206**, or base station **210**, or by the user through a user interface (not shown in figure). Factors that may be tracked include, but are not limited to, light, sound, temperature, and humidity. These factors may be tracked over time and compared to a user's sleep over the course of a night or compared over many nights in order to track correlations with these factors and the user's sleep quantity and quality. It can also be integrated into the sleep coaching plan to provide advice.

In certain embodiments, the host computer **220** and the hosted web server **230** may be combined. This combination of host computer **220** and server **230** may be located either local to the user or at a central location geographically remote from the user. This central location may be geographically distant from any individual user but also be accessible to multiple users through, for example, an internet interface.

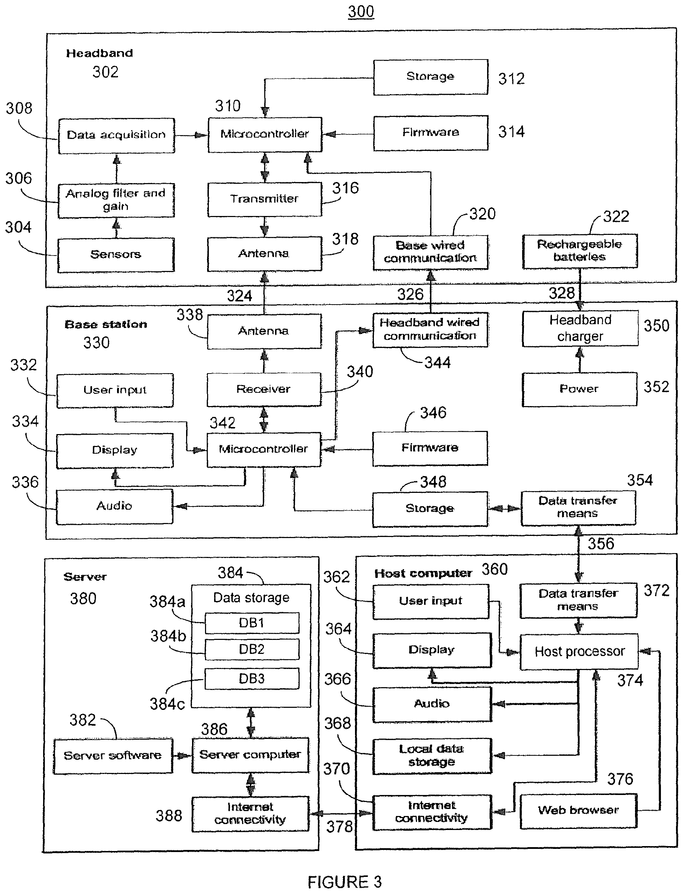

**FIG. 3** depicts a system architecture block diagram **300** for an exemplary data driven sleep coaching system, according to an illustrative embodiment of the invention. In one embodiment, the sensor module **302** may comprise a sensor housing that houses a set of dry fabric electrodes (not shown). Signals from the sensors **304** may be passed through an analog filter and gain **306**, then sent to a data acquisition module **308**. The digitized signal may then pass to a microcontroller **310** on board the electronics module (not shown). There is a battery power source **322** and on board storage **312** for the electronics module to cache data during data capture. Software running within the microcontroller **310** breaks up the stream of incoming data into data packets, and sends it wirelessly to the base station **330** via a radio frequency transmitter **316** connected to an antenna **318**. In certain embodiments, there may be a wired communication module **320** that allows the headband to communicate with the base station through headband wired communication module **344**. There may also be a headband charging module **350** that allows the headband **302** to be charged by the base station **330**.

In one embodiment, the first data transfer mechanism **324** may be implemented as a wireless connection. The packetized data may be sent wirelessly to the base station **330**, which may be received via a radio frequency receiver **340** connected to an antenna **338**. For example, an unregulated, 2.4GHz frequency band may be used with a proprietary protocol for data transmission.

In one embodiment, these packets are sent to a microcontroller **342** on board the base station **330**. The base station **330** may have user input elements **332** such as buttons, and a user display **334** such as an LCD display. In certain embodiments, base station **330** may have an audio device **336** to present sounds and alerts to the user. The base station **330** may also have on board storage **348** for caching sleep data, as well as a receptacle for a removal portable memory device such as an SD card for continuous data collection over several nights (not shown). The power source **352** of base station **330** may be based on batteries, either nonrechargeable or rechargeable, or based on power from a wall plug. The received packets of raw EEG data may be analyzed by software running on the microcontroller **342**, such as a sensor data analysis software module (not shown). The sensor data analysis software module may break the EEG data up into frequency bands and then into sleep stages. Additional sleep data may calculated by the microcontroller **342** and stored in on-board storage **348**.

In certain embodiments, the base station **330** may function as a standard alarm clock with a wake algorithm that is optionally keyed to an optimal wake theory. Sleep science indicates that the optimal time to wake a user from sleep is during REM or light sleep. Waking a user during deep sleep may result in excessive sleep inertia. The base station has access to sleep data collected throughout the night, and is therefore optionally able to sound an alarm during an optimal wakeup window given a user-specified latest wake time. An optional backup battery (not shown) may be used to guarantee that the alarm clock keeps its time even in the event of a power outage or a brownout event.

In certain embodiments, data connection **356** may comprise the physical transfer of a removable portable memory device (not shown). The removable portable memory device, such as an SD card, may be used to transfer nights of data to a host computer **360** connected to a data transfer means **372** such as a card reader. In certain embodiments, the sleep coaching program may be implemented as a hosted web based application, where the actual algorithm runs on a processor such as server computer **386** in remotely located server **380**, and the output may be presented to the user on a web browser **376**. The data may be uploaded to the remotely located server **380** over an internet connection **378**. The data may be stored and backed up on data storage **384** located on the server **380**. In certain embodiments, the first computer memory database **384a** may store user behavior and characteristics data, the second computer memory database **384b** may store sleep-related data, and the third computer memory database **384c** may store sleep-related advice. In certain embodiments, one or more of these databases may also be located in base station **330**, host computer **360**, or elsewhere. A hosted, web based application **382** running on a processor such as server computer **386** in the server **380** may incorporate an implementation of the sleep coaching program algorithm. The algorithm may analyze the uploaded sleep data on a per user basis, and may generate a step by step sleep plan for the user. This plan may then be transmitted back to the host computer **360** via an internet connection **378**, and presented to the user via a web browser **376**, using standard peripherals such as a visual display **364**, auditory output **366** and a user input **362** such as a computer keyboard and keys for user interaction.

In alternate embodiments, the sleep coaching algorithm may be implemented as a standalone desktop application that runs directly on a processor such as host processor **374** in the host computer **360**. The application may present a graphical user interface to the user. Data storage and backup may be done locally on the host computer **360**.

In another embodiment, the sensor module may directly transmit raw sensor data to a host computer via a data connection means. The sensor data analysis software module may be implemented either on the host computer as a desktop application, run by host processor **374**, or implemented as a web application running on server computer **386**. In the first example, where the data analysis software module is implemented as a desktop application, raw sensor data may be analyzed and processed into sleep data that is usable by the sleep coaching program algorithm and presented to the user as well. This reduces the amount of data that needs to be uploaded via the internet and may present a faster end user workflow. In the second example, where the data analysis software module is located on the server, the raw sensor data may be transmitted over the internet to the server. The advantage of this implementation is the consolidation of analysis software on one platform which may be updated and serviced on an as needed basis without involving user input.

In yet another embodiment, the microcontroller **310** in sensor module **302** may be augmented to include the sensor data analysis software module and provide a way to upload processed sleep data to the host computer **360** via a data transfer mechanism (not shown), again eliminating the base station **330**.

Sleep Metrics

In certain embodiments, sleep metrics may be calculated by the sensor data analysis module. These sleep metrics may be saved as the sleep data for the user. Various combinations of these sleep metrics may be presented to the user, either on the display **334** of the base station **330** or as part of the GUI displayed within a web browser **376** on the host computer **360**. **FIG. 4** depicts an illustrative representation of sleep metrics presented on a display, according to an illustrative embodiment of the invention. The sleep metric shown in **FIG. 4** is a hypnogram (see below). **[Hi - Then what is it?]** The following are examples of some possible sleep metrics, and is not a comprehensive list.

Total Z

The total amount of sleep may be calculated with the following formula:

$$\text{Total sleep time (Total Z)} = \text{Time in Bed (TiB)} - \text{Time in Wake (TiW)} - \text{Time to Sleep (Time to Z)}$$

Time to Z

The time taken for the user to fall asleep may also be calculated as Time to Sleep (Time to Z).

Bed time and rise time

The clock time when a user goes to bed and when a user gets up from bed may be calculated as Bed Time and Rise Time. In one embodiment, where a physiological signal is recorded during the night, the detection of the beginning of signal collection may be used to signify bed time, and the detection of the end of signal collection may be used to signify rise time. Signal collection start and end may be defined as whether the sensors are receiving a recognizable physiological signal from a user, as opposed to white noise from the environment.

Sleep stage breakdown

The actual time spent in each stage of sleep, as well as the percentage breakdown, may also be calculated. The stages of sleep include: Wake; Rapid Eye Movement (REM); Light (includes Stages 1 and 2) and Deep (includes Stages 3 and 4). Thus the time spent in each sleep stage may be calculated as follows. The same information for the time spent in each sleep stage may be presented as a percentage of total sleep time.

- Time in Wake

- Time in REM

- Time in Light

- Time in Deep

Number of awakenings

The number of awakenings affects how a user feels when he or she gets up in the morning, and is also used as a sleep data metric.

Hypnogram

The sleep stage as a function of time for the duration of the night may be presented to the user in the form of a hypnogram, which is presented as a bar chart where the height of each bar depicts the stage of sleep. Each bar may represent a predetermined sampling duration (e.g. 5 minutes) during the night. An exemplary depiction of a hypnogram is shown in **FIG. 4.**

The ZQ

The overall sleep quality may be presented to the user as a single number, the ZQ, which takes into account both the duration of sleep, times awakened, and time spent in each stage of sleep. In an exemplary embodiment, the ZQ may be calculated with the following formula:

$$ZQ = 8.5*(\text{Total Z}) + 0.5*(\text{Time in REM}) + 1.5*(\text{Time in Deep}) - 0.5*(\text{Time in Wake}) - 0.07*(\text{Number of Awakenings})$$

Any combination of the above information may be presented on a night-by-night basis, or it can also be viewed over time by the user. For example, the user may be interested in looking at how the Total Z changes over the course of several weeks. Alternatively, the user might be interested in investigating how the breakdown of sleep stages for a night changes over time, to see if he or she is experiencing an increase in restorative sleep (REM and deep) as opposed to light sleep. The user can also be presented data not as a function of time but rather as it correlates with other data available. For example, if a user records in a journal data which shows caffeine usage that information can be presented as a function of caffeine usage and time to fall sleep.

This information may be presented in a variety of ways. **FIG. 4** shows one example, where some of the information is presented on the display of the base station. In certain embodiments, the same information may be presented in a graphical user interface (GUI) on the host computer, whether as part of a desktop application or as a web browser based application.

In certain embodiments, some of the information may be presented on the base station (e.g. night by night data and simple trend data over several nights), while more data viewing and analysis options may be available on the host computer (e.g., detailed trend analysis of sleep stages, time to Z and the like). Additional trend information may be displayed as line charts, pie charts, tables and other graphical presentations on the host computer (not shown).

**FIG. 5** depicts a high level overview of the sleep coaching program (SCP) according to an embodiment. The SCP is a program that helps users get a better night's sleep by leveraging the unique values offered by sleep data collection and analysis, coupled with an interactive online environment with a rich multimodal user interface. The basic tenets that dictate the SCP include:

- Personalization/Customization - the user should feel that the SCP caters to them as an individual.
- Simplicity - the interface should be intuitive, instructive, and informative, without overwhelming the user.
- Education - the user should learn material that will help them continue to experience the benefits of the SCP even if they end their participation.
- Scientific Integrity - the SCP should be grounded within a theoretical framework that can be supported by the scientific community, both in sleep and in behavior.
- Effectiveness - the SCP should provide users with an educational experience that empowers them to improve their lives in an effort to improve their sleep satisfaction.

In one embodiment, the sleep coaching program (SCP) may be implemented as a step-wise program. In this type of approach, the user is guided through a number of steps to improve their sleep. Within each step, the user may be given educational and instructional materials as well as clear directions on what they should do to complete the tasks within each step. In certain embodiments, a predetermined target elapsed time (e.g. 14 days) may also be set, to help pace the user through the program and to ensure some level of closure over a given period of time.

The following example, depicted in **Fig. 5**, illustrates how this type of approach may be implemented as a 4-step program **500**.

1. Profiling the user's sleep (step **502**)

The purpose of this step is to profile or categorize a user based on their lifestyle habits and sleep profile. A user should complete this process in order to get personalized feedback. The specific tasks involved in this step comprise entering pertinent information about their demographics (male or female, as well as age range) (step **504**), answering questions about their lifestyle (step **506**), and answering questions (step **508**) that describe what type of sleeper they are or would like to be and what goals they have for sleep and lifestyle satisfaction. (step **510**). In one exemplary embodiment, the user may be guided through answering key questions as part of the account sign up and/or login process. This approach has the benefit of providing the user with immediate positive reinforcement by completing the first step of the program simply by signing up for the program. This encourages the user to stay engaged in the program and improves the overall probability of success for the user.

2. Collect sleep data from a single night's sleep (step **512**)

In this step, the user is introduced to the equipment and data collection approach used in this program, which may comprise a sensor module such as a headband with adjustable straps for attaching electrodes to the forehead of the user to collect EEG data during their sleep and a base station for storing and analyzing the raw sensor data and a data connection to upload the data to a computer. The user may learn about the program and the equipment by browsing through multimedia tutorials, FAQs and other didactic materials. They are then tasked with actually going to bed while wearing the sensors and collecting the data for one night. In one embodiment, an SD card or other portable storage device may be inserted in the base station to store the sleep data for future uploading. Upon awakening, the user is encouraged to fill in a sleep diary where they record their consumption of various substances such as caffeine and alcohol, their activities (such as any rigorous exercise within two hours of bed time), and other factors that might affect the quality and quantity of their sleep.

3. Upload data and fill in a sleep diary (step **514**)

In this step, the user may upload the data using a data transfer means, and interact with relevant parts of the web interface for the sleep coaching program to review their sleep data as well as receive personalized instructions for the sleep coaching program. In one embodiment, the user may extract the SD card or portable storage device from the base station and insert it into a card reader connected to a personal computer running a web based interface for the sleep coaching program. The user may be taken through the upload process via an interactive tutorial and completes their first data upload. The user may be prompted to fill in their sleep diary for the first time.

4. Sleep workshops (step **516**)

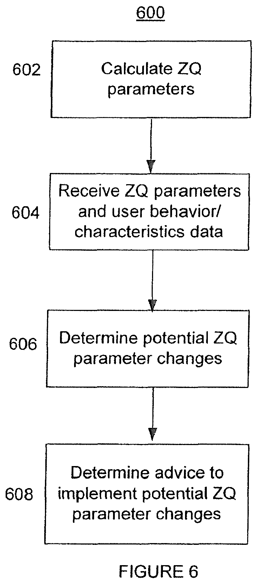

In this step, the concept of sleep workshops may be presented to the user. The sleep coaching program may use the data gathered in the previous steps to create a set of personalized advice that helps the user understand what factors affect the quality and quantity of their sleep, and what they can do to effect positive change. **FIG. 6** depicts a flowchart **600** for the creation of a set of personalized advice for improving sleep satisfaction according to an embodiment. In this embodiment, the creation of the set of personalized advice for improving sleep satisfaction may be begin by calculating the ZQ factor described above (step **602**). Once the ZQ factor is calculated, the various parameters in the ZQ equation may be examined in light of collected user behavior and characteristics data (step **606**) in order to determine parameter changes that may optimize the achievable ZQ factor (step **608**). For example, if the ratio of Time in Wake to Total Z for a particular user is lower than a particular threshold, and the user behavior data includes a particular behavior that tends to increase the time a sleeper is awake, then the system may suggest that the user reduce the particular behavior (step **608**). In certain embodiment, the user may be presented with a number of workshops, each of which is targeted to address a particular issue identified in the sleep habits and sleep data of the user. The user may choose which workshops he or she would like to follow (step **518**). For each workshop, the user may start by responding to a questionnaire that provides more in-depth questions about the topic covered in that workshop (step **520**). Then the user may be given a number of tips (e.g. four tips) (step **522**). The user should try to follow some proportion of these tips (e.g. three out of four) over the course of a predetermined interval of time (e.g. at least three nights). Data may be collected throughout the workshop, and uploaded on an ongoing basis. At the end of the workshop, a summary of the steps taken and the results achieved may be presented to the user (step **524**). Information may be presented in a multimedia fashion with text, video clips, images, audio clips, interactive quizzes and so on. The user may be prompted to collect data for a specified minimum duration of time in order to accumulate adequate baseline data to generate a customized sleep coaching program.

The user may then repeat the process for any other selected workshops where they work on a different aspect of their sleep. By the end of the workshop phase, the user should have proactively worked on trying to improve several factors that may affect their sleep, and may have data and sleep diary entries to indicate whether or not the steps taken resulted in better sleep satisfaction for the user. Once a user finishes all the steps in this program, they may continue to monitor their sleep and they may also re-engage in the stepwise program, returning to step **502**, to reassess their current state of sleep, and to come up with new data that will craft a new customized sleep coaching program with workshops targeted at improving different factors that affect their sleep at the current time. In this way, the user employs a progressive process for collecting sleep data over a subsequent period of time and getting from the system a second set of sleep advice for improving the sleep satisfaction, where the new advice is based at least in part on the sleep data associated with the second later period of time and the first set of advice given to the user.

In certain embodiments, the sleep parameters and workshops may be generated automatically by the system. In certain embodiments, a sleep expert may also provide input in the generation of sleep parameters, workshops, or otherwise contact the user.

Note that the exact number of steps and the exact contents within each step is illustrative only. The overarching invention is that this is a program that takes a user through different types of tasks in a process to educate them about sleep, collect information about how they sleep, and develop strategies to help them improve their sleep satisfaction. Other specific implementations may involve a different number of steps, different separation for the contents between each step, or different content for each step altogether. Examples of other possible steps follow (not shown):

Try a Quality-of-sleep indicator - the ZO simulator

In this step, the user may be educated about specific sleep metrics used by the sleep coaching program to gauge the quality and quantity of sleep. The user may experience an interactive simulator, where they can change certain parameters such as duration of sleep, time to fall asleep, amount of caffeine consumed within 2 hours of sleep and other such examples, and see if and how each change affects their sleep. The metrics used to gauge sleep may include: total duration of sleep; time to fall asleep; times awakened; time spent awake during the night of sleep; and a single score summarizing the quality of sleep in an easy to understand, linear metric. The quality of sleep may be presented as a single index (e.g. called the ZQ in an example implementation).

Sleep Style.

This step may be an opportunity for the user to provide more information about their particular sleep style and attitudes about sleep. This section may be composed of interactive questionnaires or quizzes, for example, so that the user can input data about their beliefs about the way they sleep. This data may be compared to physiological data that has been collected or may be later used to help determine the workshops offered to the user or the bed/rise times that are calculated to optimize the user's sleep schedule.

Recommending bed and rise time

Based on collected sleep information, a suggested optimal bed or rise time may be calculated and suggested to the user. The user may be advised to follow the bed/rise time recommendation every day, and to choose the bed and rise times such that they get an adequate amount of sleep during the night.

Final report

This step may be the conclusion of the program. A summary of the user's participation in the sleep coaching program may be provided to the user. The user may enter into a maintenance mode, much like the approach taken by weight loss programs such as Weight Watchers®. Incentives may be provided to the user to continue to use the device and website to quantify their sleep quality and to prevent any regression in the progress made to address their sleep problems.

The invention may be embodied in other specific forms without departing from the spirit or essential characteristics thereof. The foregoing embodiments are therefore to be considered in all respects illustrative, rather than limiting of the invention, and various modifications can be made by those skilled in the art without departing from the scope and spirit of the invention.

**Claims**

1. A feedback-based sleep coaching system comprising:

   a sensor (102; 202) configured to measure a physiological signal indicative of an aspect of the user's sleep;
   at least one local processor located proximately to the sensor (102; 202) and configured to process data received from the sensor (102; 202), the at least one local processor being configured for communication with a remote processor located remotely from the sensor (102; 202); and
   a user interface,
   wherein the at least one local processor is configured to:

   process the received physiological signal to obtain information of the user's sleep and generate a sleep related advice based on the obtained sleep information, or
   process the received physiological signal to obtain information of the user's sleep and forward the obtained sleep information to the remote processor for generating a sleep related advice based on the obtained sleep information and receive from the remote processor the sleep related advice, and

   wherein the user interface is configured to present the generated advice to the user.

2. The system of claim 1, wherein the at least one local processor is part of a local base station and/or host computer, and/or wherein the remote processor is part of a remote server.

3. The system of claim 1 or 2, wherein the sleep coaching system includes means of collecting at least one of:
   data representative of the user sleep data, user sleeper type, user data respective of at least one of demographic data and lifestyle data, data representative of user goals for improving sleep satisfaction, feedback to the sleep coaching system, diary data from the user representative of events in the user's life, over a selected time period, that affect the user's sleeping conditions,
   wherein the system is preferably configured to generate the advice based also on the collected data.

4. The system of any one of claims 1 to 3, wherein the presentation of advice to the user includes setting goals for the user and advice for achieving the goals.

5. The system of any one of claims 1 to 4,
   wherein the feedback-based sleep coaching system is further configured to provide feedback to the user to indicate whether the advice is improving the user's sleep,
   wherein preferably the sleep coaching feedback mechanism is configured to generate an assessment of changes in sleep quality as a function of at least one of the following: a previous measure of user sleep data and subsequent measures of user sleep data, milestones within a sleep coaching program provided to the user, a measured baseline of users sleep, a normalized baseline representative of a normative sleep measure of a predetermined population.

6. The system of any one of claims 1 to 5 wherein the sleep coaching system is configured to use data from a database to generate an interactive sleep coaching program, the data from the database including information about optimal sleep requirements as a function of age and/or gender.

7. The system of any one of claims 1 to 6, wherein the user advice comprises at least one of a recommended bedtime, risetime, or a limit on food, caffeine and/or alcohol consumption.

8. The system of any one of claims 1 to 7, wherein the sensor (102; 202) is configured to measure respiration rate and/or body movement of the user.

9. The system of any one of claims 1 to 8, wherein the sensor (102; 202) is or includes a non-contact sensor.

10. The system of any one of claims 1 to 9, wherein the system is configured to collect data of at least one environmental factor.

11. The system of any one of claims 1 to 10, wherein the system is configured to wake up the user within an optimal wakeup window.

12. The system of any one of claims 1 to 11, wherein the system is configured to display sleep stage data, retrieved from memory associated with the local processor or the remote processor, to the user in the form of a hypnogram.

13. The system of any one of claims 1 to 12, wherein the system is configured to simulate how a change In a sleep parameter is likely to change the quality of user's sleep.

14. The system of any one of claims 1 to 13, further comprising a display for presenting obtained sleep information or the advice to the user visually.

15. The system of any one of claims 1 to 14, wherein the system is arranged to, based on the obtained sleep information, calculate a single score summarizing the quality of sleep of the user and display the score to the user.

FIGURE 1A

FIGURE 1B

FIGURE 2

300

**Headband** 302

- 308 Data acquisition
- 310 Microcontroller
- 312 Storage
- 314 Firmware
- 306 Analog filter and gain
- 316 Transmitter
- 304 Sensors
- 318 Antenna
- 320 Base wired communication
- 322 Rechargeable batteries

324 326 328

**Base station** 330

- 338 Antenna
- 344 Headband wired communication
- 350 Headband charger
- 332 User input
- 340 Receiver
- 352 Power
- 334 Display
- 342 Microcontroller
- 346 Firmware
- 336 Audio
- 348 Storage
- 354 Data transfer means

356

**Server** 380

- 384 Data storage
  - 384a DB1
  - 384b DB2
  - 384c DB3
- 382 Server software
- 386 Server computer
- 388 Internet connectivity

**Host computer** 360

- 362 User input
- 372 Data transfer means
- 364 Display
- Host processor
- 366 Audio
- 374
- 368 Local data storage
- 370 Internet connectivity
- 376 Web browser
- 378

FIGURE 3

18

400

FIGURE 4

500

```
                    ┌──────────────────────┐
                    │                      │
                    │   1. Sleep Profile   │
                    │                      │
                    └──────────────────────┘
                              502

┌──────────────────┐              ┌──────────────────────┐
│  4d. Workshop    │  524         │ 1a. Demographics     │  504
│  Report          │              └──────────────────────┘
└──────────────────┘              ┌──────────────────────┐
┌──────────────────┐              │ 1b. Lifestyle        │  506
│  4c. Workshop    │  522         │ Questions            │
│  Action Plan     │              └──────────────────────┘
└──────────────────┘              ┌──────────────────────┐
┌──────────────────┐              │ 1c. Sleeper Type     │  508
│  4b. Workshop    │  520         └──────────────────────┘
│  Questionnaire   │              ┌──────────────────────┐
└──────────────────┘              │ 1d. Sleeper          │  510
┌──────────────────┐              │ Classification       │
│  4a. Workshop    │  518         └──────────────────────┘
│  selection       │
└──────────────────┘              ┌──────────────────────┐
┌──────────────────┐              │                      │
│                  │              │  2. Sleep w. Zeo     │  512
│  4. Sleep        │  516         │                      │
│  Workshops       │              └──────────────────────┘
│                  │
└──────────────────┘

             ┌──────────────────────┐
             │  3. Upload Data/     │
             │  Diary               │
             └──────────────────────┘
                      514
```

Figure 5

<u>600</u>

FIGURE 6

Europäisches
Patentamt

European
Patent Office

Office européen
des brevets

## EUROPEAN SEARCH REPORT

Application Number

EP 19 15 8530

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/096307 A1 (PHILIPS INTELLECTUAL PROPERTY [DE]; KONINKL PHILIPS ELECTRONICS NV [NL]) 14 August 2008 (2008-08-14)<br>* abstract *<br>* page 11, paragraph 3; figure 1 *<br>* page 3, paragraph 2 *<br>* page 6, paragraph 5 - page 8, paragraph 8 * | 1-15 | INV.<br>A61B5/00 |
| X | US 2004/049132 A1 (BARRON BRADFORD SCOTT [US] ET AL) 11 March 2004 (2004-03-11)<br>* paragraphs [0011], [0021], [0033], [0035] * | 1-15 | |
| X | US 2004/267565 A1 (GRUBE JAMES A [US]) 30 December 2004 (2004-12-30)<br>* abstract * | 1-15 | |
| X | US 2006/293608 A1 (ROTHMAN DANIEL [US] ET AL) 28 December 2006 (2006-12-28)<br>* paragraphs [0033], [0068] * | 1-15 | |
| X | US 2006/266356 A1 (SOTOS JOHN G [US] ET AL) 30 November 2006 (2006-11-30)<br>* paragraphs [0029], [0050] - [0054]; figure 5 * | 1-15 | TECHNICAL FIELDS SEARCHED (IPC)<br><br>A61B |
| X | US 2007/129644 A1 (RICHARDS GLENN [AU] ET AL) 7 June 2007 (2007-06-07)<br>* paragraphs [0052] - [0056] * | 1-15 | |
| X | US 2007/249952 A1 (RUBIN BENJAMIN [US] ET AL) 25 October 2007 (2007-10-25)<br>* paragraphs [0039] - [0042] * | 1-15 | |

The present search report has been drawn up for all claims

| Place of search | Date of completion of the search | Examiner |
|---|---|---|
| Berlin | 1 October 2019 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 19 15 8530

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2019

| Patent document cited in search report | Publication date | Patent family member(s) | | Publication date |
|---|---|---|---|---|
| WO 2008096307 A1 | 14-08-2008 | NONE | | |
| US 2004049132 A1 | 11-03-2004 | NONE | | |
| US 2004267565 A1 | 30-12-2004 | NONE | | |
| US 2006293608 A1 | 28-12-2006 | US 2006293608 A1 | | 28-12-2006 |
| | | WO 2005084538 A1 | | 15-09-2005 |
| US 2006266356 A1 | 30-11-2006 | US 2006266356 A1 | | 30-11-2006 |
| | | US 2013102928 A1 | | 25-04-2013 |
| US 2007129644 A1 | 07-06-2007 | AU 2005242119 A1 | | 21-06-2007 |
| | | US 2007129644 A1 | | 07-06-2007 |
| US 2007249952 A1 | 25-10-2007 | DE 102007009722 A1 | | 30-04-2008 |
| | | US D626240 S | | 26-10-2010 |
| | | US 2007249952 A1 | | 25-10-2007 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61196960 A
- US 38773009 A
- US 58619606 A
- US 11499407 B
- US 11069934 B